(19) Europäisches Patentamt
European Patent Office
Office européen des brevets

(11) Veröffentlichungsnummer: **0 216 102**
**A1**

(12) **EUROPÄISCHE PATENTANMELDUNG**

(21) Anmeldenummer: 86111075.7

(22) Anmeldetag: 11.08.86

(51) Int. Cl.⁴: C 07 D 401/04
C 07 D 231/18, A 01 N 43/56
//C07D231/44, C07D231/38

(30) Priorität: 21.08.85 DE 3529829

(43) Veröffentlichungstag der Anmeldung: 01.04.87 Patentblatt 87/14

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Jensen-Korte, Uta, Dr.
Geibelstrasse 9
D-4000 Düsseldorf 1(DE)

(72) Erfinder: Gehring, Reinhold, Dr.
Dasnöckel 49
D-5600 Wuppertal 11(DE)

(72) Erfinder: Schallner, Otto, Dr.
Noldeweg 22
D-4019 Monheim(DE)

(72) Erfinder: Stetter, Jörg, Dr.
Gellertweg 4
D-5600 Wuppertal 1(DE)

(72) Erfinder: Wroblowsky, Heinz-Jürgen, Dr.
Gladbacher Strasse 34
D-4018 Langenfeld(DE)

(72) Erfinder: Becker, Benedikt, Dr.
Metzkausener Strasse 14
D-4020 Mettmann(DE)

(72) Erfinder: Stendel, Wilhelm, Dr.
In den Birken 55
D-5600 Wuppertal 1(DE)

(72) Erfinder: Homeyer, Bernhard, Dr.
Obere Strasse 28
D-5090 Leverkusen 3(DE)

(72) Erfinder: Behrenz, Wolfgang, Dr.
Untergründemich 14
D-5063 Overath(DE)

(54) **1-Arylpyrazole.**

(57) Es wurden neue 1-Aryl-pyrazole der allgemeinen Formel

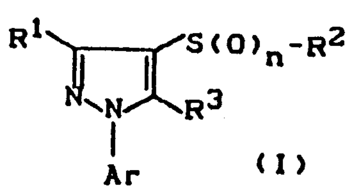

(I)

bereitgestellt, worin
$R^1$ für Wasserstoff, Alkyl oder Halogenalkyl steht,
$R^2$ für Alkyl, Halogenalkyl oder für jeweils gegebenenfalls substituiertes Aralkyl oder Aryl steht,
$R^3$ für Wasserstoff oder Halogen steht,
Ar für gegebenenfalls substituiertes Phenyl oder Pyridyl steht, und
n für eine Zahl 0, 1 oder 2 steht.
Die neuen Verbindungen weisen überragende Eigenschaften als Schädlingsbekämpfungsmittel, insbesondere als Insektizide und Akarizide auf.

0216102

BAYER AKTIENGESELLSCHAFT
Konzernverwaltung RP
Patentabteilung

5090 Leverkusen, Bayerwerk
Er/ABc
Ia

## 1-Arylpyrazole

Die Erfindung betrifft neue 1-Arylpyrazole, ein Verfahren zu ihrer Herstellung und ihre Verwendung als Schädlingsbekämpfungsmittel, insbesondere als Insektizide und Akarizide.

Es ist bereits bekannt, daß bestimmte Pyrazol-Derivate, wie beispielsweise das 1-Cyclohexyl-5-[N,N-(dimethyl)-carbamoyloxy]-3-methylthiomethyl-pyrazol oder das 1-Cyclohexyl-5-[N,N-(dimethyl)-carbamoyloxy]-3-methylsulfonyl methyl-pyrazol insektizide, nematizide und fungizide Wirkung besitzen.

Die Wirkungshöhe bzw. Wirkungsdauer dieser Verbindungen sind jedoch, insbesondere bei bestimmten Insekten oder bei niedrigen Anwendungskonzentrationen nicht immer in allen Anwendungsbereichen völlig zufriedenstellend.

Es wurden nun neue 1-Aryl-pyrazole der allgemeinen Formel (I),

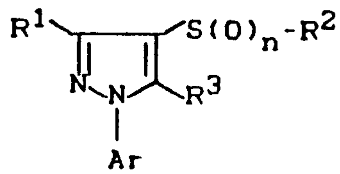

(I)

in welcher

$R^1$ für Wasserstoff, Alkyl oder Halogenalkyl steht,

$R^2$ für Alkyl, Halogenalkyl oder für jeweils gegebenenfalls substituiertes Aralkyl oder Aryl steht,

$R^3$ für Wasserstoff oder Halogen steht,

Ar für gegebenenfalls substituiertes Phenyl oder Pyridyl steht,

und

n für eine Zahl 0, 1 oder 2 steht,

gefunden.

Weiterhin wurde gefunden, daß man die neuen 1-Aryl-pyrazole der allgemeinen Formel (I),

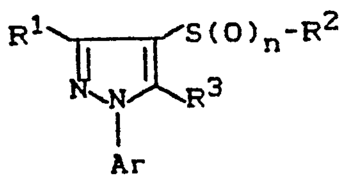

(I)

in welcher

$R^1$ für Wasserstoff, Alkyl oder Halogenalkyl steht,

$R^2$ für Alkyl, Halogenalkyl oder für jeweils gegebenenfalls substituiertes Aralkyl oder Aryl steht,

$R^3$ für Wasserstoff oder Halogen steht,

Ar für gegebenenfalls substituiertes Phenyl oder Pyridyl steht,

und

n für eine Zahl 0,1 oder 2 steht,

erhält, wenn man 5-Amino-1-aryl-pyrazole der Formel (II),

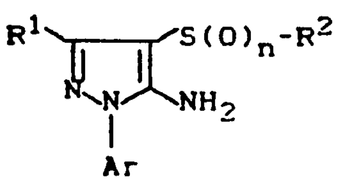

(II)

in welcher

$R^1$, $R^2$, Ar und n die oben angegebene Bedeutung haben,

mit einem anorganischen oder organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

Schließlich wurde gefunden, daß die neuen 1-Aryl-pyrazole der allgemeinen Formel (I) insektizide und akarizide Eigenschaften besitzen.

Überraschenderweise zeigen die erfindungsgemäßen 1-Arylpyrazole der allgemeinen Formel (I) eine erheblich bessere insektizide und akarizide Wirksamkeit, als die aus dem Stand der Technik bekannten Pyrazol-Derivate, wie beispielsweise das 1-Cyclohexyl-5-[N,N-(dimethyl)-carbamoyloxy]-3-methylthiomethyl-pyrazol oder das 1-Cyclohexyl-5-[N,N-(dimethyl)-carbamoyloxy]-3-methylsulfonylmethyl-pyrazol, welches chemisch und wirkungsmäßig naheliegende Verbindungen sind.

Die erfindungsgemäßen 1-Aryl-pyrazole sind durch die Formel (I) allgemein definiert. Bevorzugt sind Verbindungen der Formel (I), bei welchen

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht oder für jeweils gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges

oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für Wasserstoff, Fluor, Chlor, Brom oder Iod steht,

Ar für gegebenenfalls einfach oder mehrfach, gleich oder verschieden substituiertes Phenyl, 2-Pyridyl, 3-Pyridyl oder 4-Pyridyl steht, wobei als Substituenten jeweils in Frage kommen:
Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$,

wobei

$R^4$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

m für eine Zahl 0, 1 oder 2 steht und

n für eine Zahl 0, 1 oder 2 steht.

Besonders bevorzugt sind 1-Arylpyrazole der Formel (I), bei welchen

$R^1$ für Wasserstoff, Methyl, Ethyl, n- oder i-Propyl und Trifluormethyl steht,

$R^2$ für Methyl, Ethyl, n- oder i-Propyl, n-, i-, s- oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl oder für jeweils gegebenenfalls einfach bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Wasserstoff, Fluor, Chlor oder Brom steht,

Ar für gegebenenfalls ein- bis fünffach, gleich oder verschieden substituiertes Phenyl oder für jeweils gegebenenfalls ein- bis vierfach gleich oder verschieden substituiertes 2-Pyridyl oder 4-Pyridyl steht, wobei als Phenyl- bzw.Pyridylsubstituenten jeweils in Frage kommen: Cyano, Nitro, Fluor, Chlor, Brom, Iod, Methyl, Ethyl, n- und i-Propyl, n-, i-,

s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,

wobei

$R^4$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht,

m für eine Zahl 0, 1 oder 2 steht und

n für eine Zahl 0, 1 oder 2 steht.

Im einzelnen seien außer den bei den Herstellungsbeispielen genannten Verbindungen die folgenden 1-Arylpyrazole der allgemeinen Formel (I) genannt:

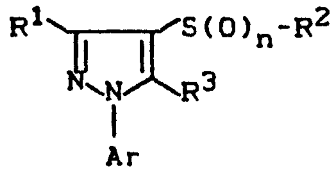

(I)

| $R^1$ | $R^2$ | $R^3$ | n | Ar |
|---|---|---|---|---|
| H | $-CF_2Cl$ | H | 0 | 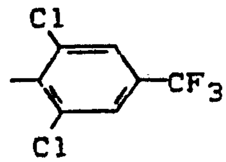 |
| H | $-CF_2Cl$ | H | 1 | 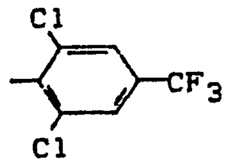 |
| H | $-CF_2Cl$ | H | 2 | 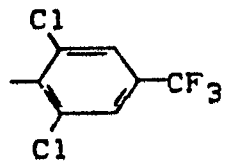 |
| H | $-CF_2Cl$ | Cl | 0 | 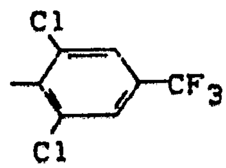 |
| H | $-CF_2Cl$ | Cl | 1 | 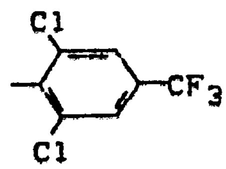 |
| H | $-CF_2Cl$ | Cl | 2 | 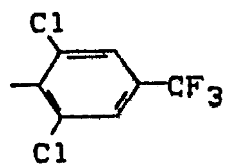 |
| H | $-CF_2Cl$ | Br | 0 | 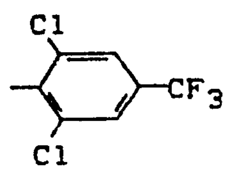 |

| $R^1$ | $R^2$ | $R^3$ | n | Ar |
|---|---|---|---|---|
| H | $-CF_2Cl$ | Br | 1 | 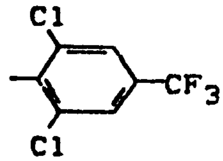 |
| H | $-CF_2Cl$ | Br | 2 | 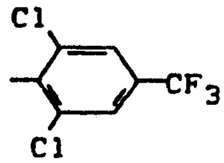 |
| $CH_3$ | $-CF_2Cl$ | H | 0 | 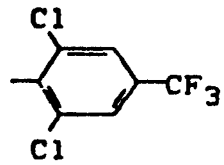 |
| $CH_3$ | $-CF_2Cl$ | H | 1 | 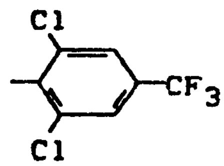 |
| $CH_3$ | $-CF_2Cl$ | H | 2 | 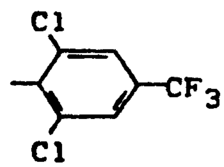 |
| $CH_3$ | $-CF_2Cl$ | Cl | 0 | 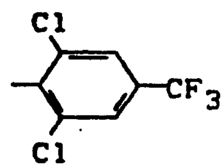 |
| $CH_3$ | $-CF_2Cl$ | Br | 0 | 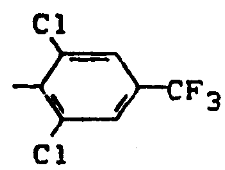 |

| $R^1$ | $R^2$ | $R^3$ | n | Ar |
|---|---|---|---|---|
| H | $-CF_3$ | H | 0 | 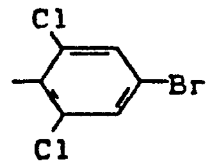 |
| H | $-CF_3$ | H | 2 | 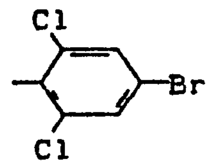 |
| H | $-CF_3$ | Cl | 0 | 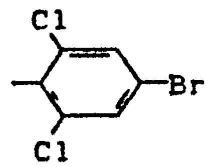 |
| H | $-CF_3$ | Cl | 2 | 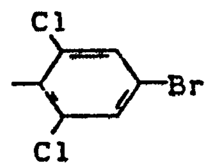 |
| H | $-CF_3$ | Br | 0 | 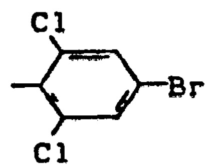 |
| H | $-CF_3$ | Br | 2 | 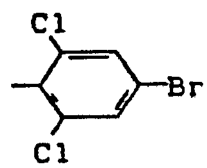 |
| H | $-CF_2Cl$ | H | 0 | 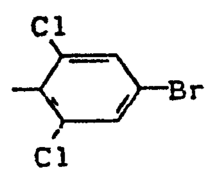 |

| $R^1$ | $R^2$ | $R^3$ | n | Ar |
|---|---|---|---|---|
| H | $-CF_2Cl$ | H | 1 | 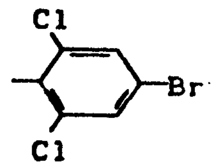 |
| H | $-CF_2Cl$ | H | 2 | 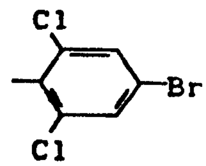 |
| H | $-CF_2Cl$ | Cl | 0 | 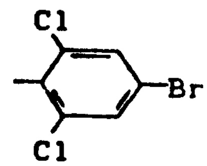 |
| H | $-CF_2Cl$ | Cl | 2 | 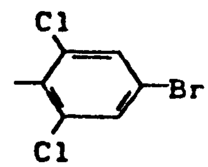 |
| H | $-CF_2Cl$ | Br | 2 | 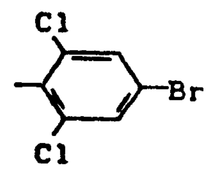 |
| $CH_3$ | $-CF_3$ | Cl | 0 | 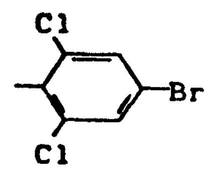 |
| $CH_3$ | $-CF_3$ | H | 0 | 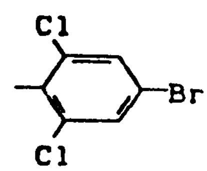 |

| $R^1$ | $R^2$ | $R^3$ | n | Ar |
|---|---|---|---|---|
| $CH_3$ | $-CF_3$ | H | 1 | 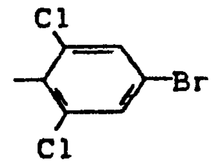 |
| $CH_3$ | $-CF_3$ | H | 2 | 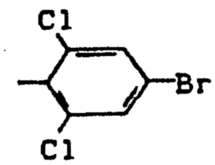 |
| $CH_3$ | $-CF_2Cl$ | H | 0 | 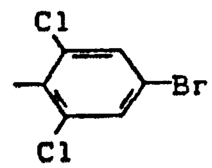 |
| $CH_3$ | $-CF_2Cl$ | H | 1 | 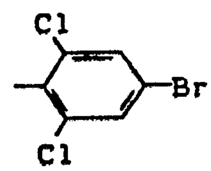 |
| $CH_3$ | $-CF_2Cl$ | H | 2 | 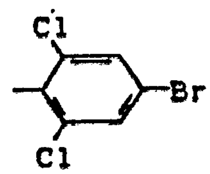 |
| $CH_3$ | $-CF_2Cl$ | Cl | 0 | 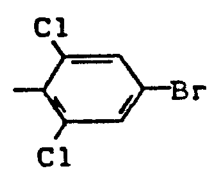 |
| $CH_3$ | $-CF_2Cl$ | Br | 0 | 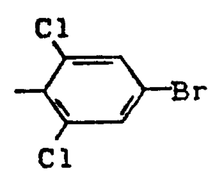 |

| $R^1$ | $R^2$ | $R^3$ | n | Ar |
|---|---|---|---|---|
| $CH_3$ | $-CCl_2F$ | H | 0 | 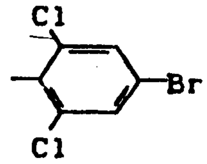 |
| $CH_3$ | $-CCl_2F$ | H | 1 | 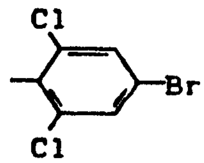 |
| $CH_3$ | $-CCl_2F$ | H | 2 | 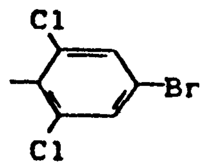 |
| $CH_3$ | $-CCl_2F$ | Cl | 0 | 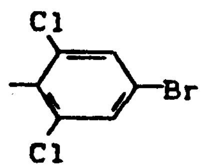 |
| $CH_3$ | $-CCl_2F$ | Br | 0 | 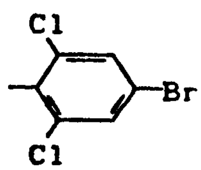 |
| H | H | H | 0 | 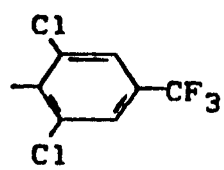 |
| H | $CH_3$ | H | 1 | 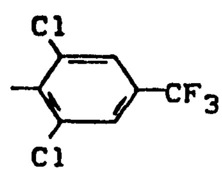 |

| $R^1$ | $R^2$ | $R^3$ | n | Ar |
|---|---|---|---|---|
| H | $CH_3$ | H | 2 | 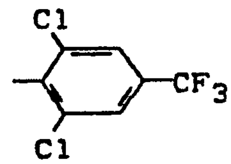 |
| H | $CH_3$ | Cl | 0 | 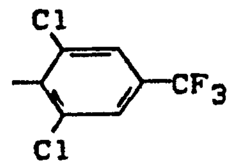 |
| H | $CH_3$ | Br | 0 | 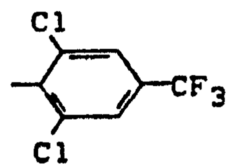 |
| H | $CH_3$ | Cl | 0 | 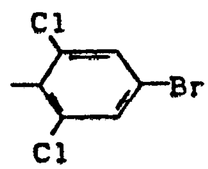 |
| H | $CH_3$ | Br | 0 | 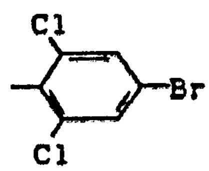 |

Verwendet man beispielsweise 5-Amino-4-dichlorfluormethylsulfenyl-1-(2,6-dichlor-4-trifluormethyl-phenyl)-pyrazol als Ausgangsstoff und Natriumnitrit/Bromwasserstoffsäure als Reagenzien, so läßt sich der Reaktionsablauf des erfindungsgemäßen Verfahrens durch das folgende Formelschema darstellen:

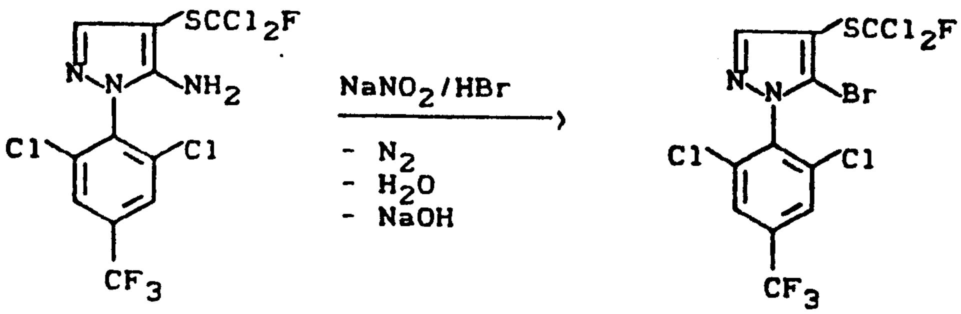


Die zur Durchführung des erfindungsgemäßen Verfahrens als Ausgangsstoffe benötigten 5-Amino-1-aryl-pyrazole sind durch die Formel (II) allgemein definiert. In dieser Formel (II) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten und Indizes genannt wurden.

Die 5-Amino-1-aryl-pyrazole der Formel (II) sind noch nicht bekannt. Sie sind jedoch Gegenstand der eigenen vorgängigen deutschen Patentanmeldungen P 3 402 308 (Le A 22 853) und DE-P 3 517 843 (Le A 23 753).

Man erhält sie, wenn man

4-Thiocyanato-5-aminopyrazole der allgemeinen Formel (III),

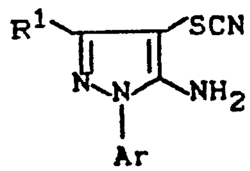

(III)

in welcher

$R^1$ und Ar die oben angegebene Bedeutung haben, oder

Bis-(pyrazolyl)-disulfide der Formel (IV),

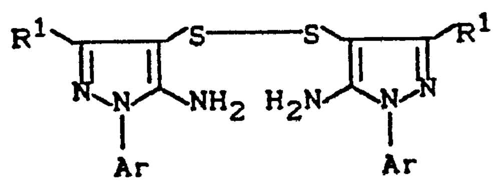

(IV)

in welcher

$R^1$ und Ar die oben angegebene Bedeutung haben,

mit Halogeniden der Formel (V)

$$R^2\text{-Hal}^1 \qquad (V)$$

in welcher

$R^2$ die oben angegebene Bedeutung hat und

$Hal^1$ für Halogen, insbesondere für Chlor, Brom oder Iod steht,

gegebenenfalls in Gegenwart eines Verdünnungsmittels wie beispielsweise Methanol oder Ethanol sowie gegebenenfalls in Gegenwart eines Reduktionsmittels wie beispielsweise Natriumborhydrid oder Natriumdithionit und gegebenenfalls in Gegenwart einer Base, wie beispielsweise Natriumhydroxid oder Kaliumcarbonat bei Temperaturen zwischen 20°C und 90°C umsetzt, oder wenn man

4-unsubstituierte 5-Amino-pyrazole der Formel (VI)

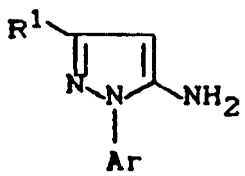

(VI)

in welcher

$R^1$ und Ar die oben angegebene Bedeutung haben,

mit Sulfenylhalogeniden der Formel (VII),

0216102

$R^2$-S-$Hal^2$ (VII)

in welcher

$R^2$ die oben angegebene Bedeutung hat und

$Hal^2$ für Halogen, insbesondere für Fluor, Chlor, Brom oder Iod steht,

gegebenenfalls in Gegenwart eines Verdünnungsmittels wie beispielsweise Dichlormethan und gegebenenfalls in Gegenwart eines Säurebindemittels wie beispielsweise Pyridin bei Temperaturen zwischen $0^\circ C$ und $50^\circ C$ umsetzt, und gegebenenfalls anschließend die so erhältlichen 5-Aminopyrazole der Formel (IIa),

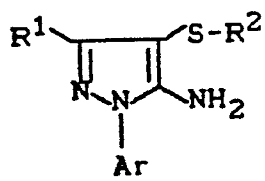

(IIa)

in welcher

$R^1$ , $R^2$ und Ar die oben angegebene Bedeutung haben,

mit Oxidationsmitteln der Formel (VIII),

R-O-OH (VIII)

in welcher

R für Wasserstoff oder für jeweils gegebenenfalls substituiertes Alkanoyl oder Aroyl, vorzugsweise für Wasserstoff, für Acetyl, für Propionyl, für Trifluoracetyl oder für gegebenenfalls substituiertes Benzoyl wie beispielsweise 3-Chlorbenzoyl oder 4-Nitrobenzoyl steht,

gegebenenfalls in Gegenwart eines Verdünnungsmittels wie beispielsweise Dichlormethan, gegebenenfalls in Gegenwart eines Katalysators wie beispielsweise Ammoniummolybdat sowie gegebenenfalls in Gegenwart eines Säurebindemittels wie beispielsweise Natriumcarbonat oder Natriumbicarbonat bei Temperaturen zwischen 0° C und 50° C am Schwefel der Sulfenylgruppe in 4-Position des Pyrazolringes oxidiert.

Die 4-Thiocyanato-5-aminopyrazole der Formel (III) sind teilweise bekannt (vgl. z.B. Farmaco Ed. Sci. 38, 274-282 [1983]). Man erhält sie beispielsweise, wenn man 4-unsubstituierte 5-Aminopyrazole der Formel (VI),

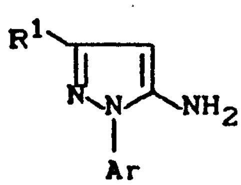

(VI)

in welcher

$R^1$ und Ar die oben angegebene Bedeutung haben,

mit Ammoniumthiocyanat in Gegenwart von Brom und Essigsäure bei Temperaturen zwischen -20° C und +20° C umsetzt.

Die Bis-(pyrazolyl)-disulfide der Formel (IV) sind noch nicht bekannt. Man erhält sie, wenn man die oben beschriebenen 4-Thiocyanato-5-amino-pyrazole der Formel (III) mit wässriger Salzsäure, gegebenenfalls in Gegenwart eines Verdünnungsmittels, wie beispielsweise Ethanol bei Temperaturen zwischen 20° C und 120° C umsetzt.

Die Halogenide der Formel (V) sind allgemein bekannte Verbindungen der organischen Chemie.

Die 4-unsubstituierten 5-Aminopyrazole der Formel (VI) sind teilweise bekannt (vgl. z.B. J. Org. Chem. 36, 2972-2974 [1971] oder J. Heterocyclic Chemistry 7, 345-349 [1970]; C.A. 62: 13137c).

Man erhält sie beispielsweise, wenn man Arylhydrazine der Formel (IX),

$$Ar\text{-}NH\text{-}NH_2 \qquad (IX)$$

in welcher

Ar die oben angegebene Bedeutung hat,

mit Acrylnitrilderivaten der Formel (X),

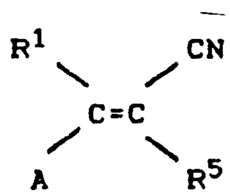

(X)

in welcher

$R^1$ die oben angegebene Bedeutung hat,

$R^5$ für Wasserstoff oder Alkoxycarbonyl steht und

A für Halogen, Hydroxy, Alkoxy, Amino oder Dialkylamino steht,

entweder zunächst in einer 1.Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen -20° C und +20° C umsetzt zu den Arylhydrazinderivaten der Formel (XI),

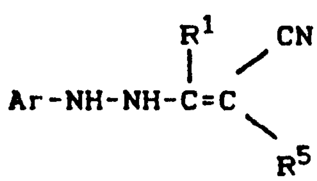

(XI)

in welcher

Ar, $R^1$ und $R^5$ die oben angegebene Bedeutung haben,

und diese in einer 2.Stufe, gegebenenfalls in Gegenwart eines Verdünnungsmittels wie beispielsweise Ethylenglykolmonoethylether bei Temperaturen zwischen +50° C und +150° C cyclisiert, oder in einem Reaktionsschritt ohne Isolierung der Zwischenstufe der Formel (XI), gegebenenfalls in Gegenwart eines Verdünnungsmittels wie beispielsweise Ethylenglykolmonoethylether bei Temperaturen zwischen +50° C und +150° C direkt cyclisiert zu den 5-Aminopyrazolen der Formel (VIa),

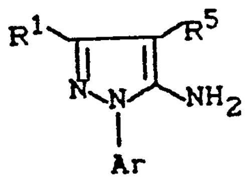

(VIa)

in welcher

$R^1$, $R^5$ und Ar die oben angegebene Bedeutung haben,

und in dem Fall, wo $R^5$ für Alkoxycarbonyl steht, die Verbindungen der Formel (VIa), in allgemein üblicher Art und Weise, gegebenenfalls in Gegenwart eines Verdünnungsmittels, wie beispielsweise Ethanol oder Isopropanol sowie gegebenenfalls in Gegenwart eines Katalysators wie beispielsweise Bromwasserstoffsäure, bei Temperaturen zwischen 50°C und 150°C verseift und decarboxyliert.

Die Arylhydrazine der Formel (IX) sind bekannt (vgl. z.B. US-PS 4 127 575; US-PS 3 609 158; DE-OS 2 558 399; J. Chem. Soc. C, 1971, 167-174) oder lassen sich nach prinzipiell bekannten Verfahren in einfacher analoger Weise herstellen (vgl. Houben-Weyl "Methoden der organischen Chemie" Band X, 2 S.203, Thieme Verlag Stuttgart 1967).

Die Acrylnitrilderivate der Formel (X) sind allgemein bekannte Verbindungen der organischen Chemie.

Die Sulfenylhalogenide der Formel (VII) sind allgemein bekannte Verbindungen der organischen Chemie.

Die Oxidationsmittel der Formel (VIII) sind ebenfalls allgemein bekannte Verbindungen der organischen Chemie.

Als Verdünnungsmittel zur Durchführung des erfindungsgemäßen Verfahrens kommen alle üblicherweise für derartige Diazotierungsreaktionen üblichen Lösungsmittel in Frage. Vorzugsweise verwendet man Halogenkohlenwasserstoffe wie Chloroform oder Bromoform oder wässrige Säuren wie beispielsweise Halogenwasserstoffsäuren oder Schwefelsäure, wobei die Säurekomponente gleichzeitig als Reagenz und/oder als Reaktionshilfsmittel fungiert. Bei der Verwendung von Bromoform als Verdünnungsmittel erhält man in der Regel die entsprechenden 5-Brom-pyrazole, wobei das Bromoform gleichzeitig als Verdünnungsmittel und als Reagenz fungiert.

Die entsprechende Reaktion in Gegenwart von Chloroform als Verdünnungsmittel ergibt im allgemeinen eine Mischung von 5-Chlor-pyrazolverbindungen der Formel (I) und den analogen reduzierten Verbindungen der Formel (I) die in der 5-Position des Pyrazolringes einen Wasserstoffrest tragen. Diese Mischungen lassen sich destillativ auftrennen.

Das erfindungsgemäße Verfahren wird in Gegenwart eines anorganischen oder organischen Nitrits durchgeführt. Als solche kommen alle üblicherweise für derartige Diazotierungsreaktionen üblichen Nitritverbindungen in Frage. Besonders bevorzugt verwendet man Alkalimetallnitrite, wie beispielsweise Natriumnitrit oder Alkylnitrite wie beispielsweise t-Butylnitrit.

Das erfindungsgemäße Verfahren wird gegebenenfalls in Gegenwart einer Halogenwasserstoffsäure durchgeführt. In diesem Fall erhält man als Reaktionsprodukte 1-Aryl-pyrazole der Formel (I), bei welchen der Rest $R^3$ für einen Halogenrest steht, der dem Anion der verwendeten Halogenwasserstoffsäure entspricht. Vorzugsweise verwender man jeweils wässrige Lösungen der Fluorwasserstoffsäure, Chlorwasserstoffsäure, Bromwasserstoffsäure oder Iodwasserstoffsäure.

Das erfindungsgemäße Verfahren wird üblicherweise in Gegenwart eines Reaktionshilfsmittels durchgeführt. Als solche kommen insbesondere starke Mineralsäuren wie Schwefelsäure oder Phosphorsäure oder die oben aufgeführten Halogenwasserstoffsäuren in Frage, die in diesem Fall gleichzeitig als Reagenz und als Katalysator wirken.

Das erfindungsgemäße Verfahren kann gegebenenfalls in Gegenwart eines geeigneten Reduktionsmittels durchgeführt werden. In diesem Fall erhält man als Reaktionsprodukte 1-Aryl-pyrazole der Formel (I), bei welchen der Rest $R^3$ für Wasserstoff steht. Als Reduktionsmittel verwendet man in diesen Fällen besonders bevorzugt unterphosphorige Säure ($H_3PO_2$).

Die Reaktionstemperaturen können bei der Durchführung des erfindungsgemäßen Verfahrens in einem größeren Bereich variiert werden. Im allgemeinen arbeitet man bei Temperaturen zwischen -30° C und +60° C, vorzugsweise bei Temperaturen zwischen -20° C und +40° C.

Zur Durchführung des erfindungsgemäßen Verfahrens setzt man pro Mol an 5-Amino-1-aryl-pyrazol der Formel (II) im allgemeinen 1,0 bis 1,8 Mol an Nitrit, gegebenenfalls 1,0 bis 20,0 Mol, vorzugsweise 1,0 bis 10,0 Mol an Halogenwasserstoffsäure, gegebenenfalls 1,0 bis 50,0 Mol, vorzugsweise 1,0 bis 20,0 Mol an Reduktionsmittel und gegebenenfalls 1,0 bis 20,0 Mol, vorzugsweise 1,0 bis 10,0 Mol an als Reaktionshilfsmittel verwendete Mineralsäure ein.

Dabei setzt man üblicherweise der Reaktionsmischung bestehend aus 5-Amino-1-aryl-pyrazol der Formel (II), Mineralsäure, Verdünnungsmittel und Halogenwasserstoffsäure bzw. Reduktionsmittel das Nitrit in kleinen Portionen gegebenenfalls in geeignetem Verdünnungsmittel gelöst zu.

Die Aufarbeitung und Isolierung der Reaktionsprodukte der Formel (I) erfolgt nach üblichen Methoden z.B durch Abfiltrieren von kristallinen Produkten oder durch Extraktion mit einem geeigneten organischen Lösungsmittel. Die Identifizierung erfolgt durch Schmelzpunkt oder Protonen-Kernresonanz-Spektrum.

Erfindungsgemäße Verbindungen der Formel (Ia),

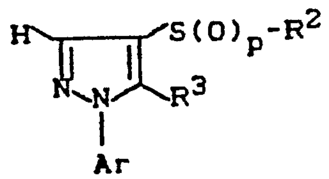

(Ia)

in welcher

$R^2$ und Ar die oben angegebene Bedeutung haben, und

p für eine Zahl 1 oder 2 steht,

erhält man alternativ auch aus den erfindungsgemäßen Verbindungen der Formel (Ib),

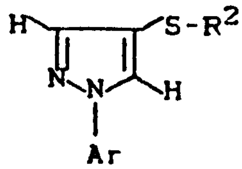

(Ib)

in welcher

$R^2$ und Ar die oben angegebene Bedeutung haben,

wenn man in üblicher Art und Weise mit Oxidationsmitteln der Formel (VIII),

R-O-OH (VIII)

in welcher

R für Wasserstoff oder für jeweils gegebenenfalls substituiertes Alkanoyl oder Aroyl, insbesondere für Wasserstoff, für Acetyl, Propionyl, Trifluoracetyl oder für gegebenenfalls substituiertes Benzoyl, wie beispielsweise 3-Chlorbenzoyl oder 4-Chlorbenzoyl steht,

gegebenenfalls in Gegenwart eines Verdünnungsmittels wie beispielsweise Dichlormethan und gegebenenfalls in Gegenwart eines Katalysators wie beispielsweise Ammoniummolybdat und gegebenenfalls in Gegenwart eines Säurebindemittels wie beispielsweise Natriumcarbonat oder Natriumbicarbonat bei Temperaturen zwischen 0° C und 50° C am Schwefel der Sulfenylgruppe in 4-Position des Pyrazolringes oxidiert (vgl. auch Herstellungsbeispiele).

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus

spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räubemilben, Laufmilben, Fliegen (stechend und leckend), parasitierende Fliegenlarven, Läuse, Haarlinge, Federlinge, Flöhe.
Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe insektizide und akarizide Wirksamkeit aus. Sie lassen sich mit besonders gutem Erfolg gegen pflanzenschädigende Insekten, wie beispielsweise gegen die Larven der Meerrettichblattkäfer (Phaedon cochleariae) sowie gegen pflanzenschädigende Milben, wie beispielsweise gegen die gemeine Spinnmilbe (Tetranychus urticae) einsetzen. Daneben eignen sie sich auch hervorragend zur Bekämpfung von Bodeninsekten und lassen sich beispielsweise zur Bekämpfung von Phorbia antiqua Maden einsetzen.

Außerdem besitzen die erfindungsgemäßen Wirkstoffe eine hohe Wirkung gegen Hygiene- und Vorratsschädlinge und lassen sich beispielsweise zur Bekämpfung der Stubenfliege (Musca domestica), zur Bekämpfung des gemeinen Kornkäfers (Sitophilus granarius) oder zur Bekämpfung der Madeiraschabe (Leucophaea maderae) einsetzen. Darüber hinaus lassen sich die erfindungsgemäßen Wirkstoffe mit besonders gutem Erfolg zur Bekämpfung von parasitisch lebenden Warmblüterschädlingen wie beispielsweise gegen die Larven der Goldfliege (Lucilia cuprina) oder gegen Stechfliegen (Stomoxys calcitrans) einsetzen. Daneben besitzen die erfindungsgemäßen Wirkstoffe in entsprechenden Aufwandmengen auch eine gute fungizide Wirksamkeit und lassen sich beispielsweise zur Bekämpfung von Botrytis- und Venturia-Arten einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage:

z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden.

Herstellungsbeipiele:

Beispiel 1:

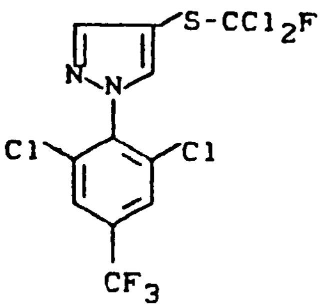

15 g (0,035 Mol) 5-Amino-4-dichlorfluormethylmercapto-1-(2,6-dichlor-4-trifluormethylphenyl)-pyrazol werden in einer Mischung aus 100 ml Eisessig und 35 ml 85 %iger Phosphorsäure gelöst und bei $0^{\circ}$ C mit einer Lösung von 2,9 g (0,042 Mol) Natriumnitrit in 22 ml 96 %iger Schwefelsäure versetzt. Es wird eine Stunde bei $0^{\circ}$ C gerührt und dann bei dieser Temperatur tropfenweise mit 50 ml 50 %iger hypophosphoriger Säure versetzt. Nach beendeter Zugabe wird drei Stunden bei 0-$10^{\circ}$ C gerührt, danach mit 200 ml Dichlormethan extrahiert. Die organische Phase wird abgetrennt, mit Natriumhydrogencarbonat und Kochsalzlösung gewaschen und über Magnesiumsulfat getrocknet. Nach Abdampfen des Lösungsmittels im Vakuum erhält man 14,2 g eines gelben Öls, das säulenchromatographisch mit Kieselgel als Trägermittel und Methylenchlorid als Laufmittel gereinigt wird.

Man erhält 5,2 g (36 % der Theorie) an 4-Dichlor-fluormethansulfenyl-1-(2,6-dichlor-4-trifluormethyl-phenyl)-pyrazol vom Schmelzpunkt 58°C.

Herstellung der Ausgangsverbindung

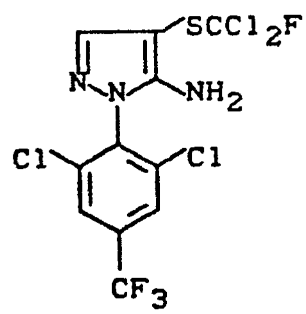

10 g (0,034 Mol) 5-Amino-1-(2,6-dichlor-4-trifluormethyl)-pyrazol werden in 50 ml Eisessig gelöst und bei Raumtemperatur tropfenweise mit 6,1 g (0,036 Mol) Dichlorfluormethansulfenylchlorid versetzt. Die Temperatur steigt bis auf ca. 40°C an. Die Reaktionsmischung wird

2 Stunden gerührt und danach in eine Mischung aus 200 ml Wasser und 50 ml Dichlormethan eingetragen. Die organische Phase wird abgetrennt, die wässrige mit zweimal 20 ml Dichlormethan extrahiert. Die vereinigten organischen Phasen werden nacheinander mit Natriumhydrogencarbonat- und Kochsalzlösung gewaschen, über Magnesiumsulfat getrocknet und im Vakuum eingeengt

Man erhält 13,6 g (94 % der Theorie) an 5-Amino-4-dichlorfluormethansulfenyl-1-(2,6-dichlor-4-trifluormethyl-phenyl)-pyrazol vom Schmelzpunkt 100° C-103° C.

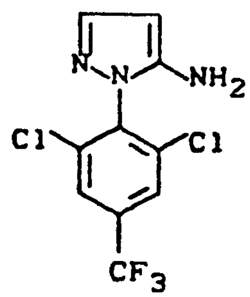

24,5 g (0,1 Mol) 2,6-Dichlor-4-trifluormethylphenylhydrazin und 20 mg Ethylendiamin-tetraessigsäure- Dinatriumsalz (=Titriplex III) in 150 ml Methanol werden bei Rückflußtemperatur tropfenweise mit 25 ml (27,6 g/ 0,3 Mol) 2-Chlor-acrylnitril versetzt. Nach beendeter Zugabe erhitzt man weitere 8 Stunden auf Rückflußtemperatur, tropft dann 9 ml (0,16 Mol) 96 %ige Schwefelsäure zu und erhitzt weitere 6 Stunden auf Rückflußtemperatur.

Die abgekühlte Reaktionsmischung wird mit 33,5 g (0,3 Mol) wasserfreiem Natriumcarbonat versetzt. Nach 4 Stunden entfernt man das Lösungsmittel im Vakuum, nimmt den Rückstand in 500 ml Wasser auf und rührt 10 Stunden bei Raumtemperatur. Man filtriert den ausgefallenen Niederschlag ab, wäscht mit Wasser nach und trocknet im Vakuum bei 50° C.

Man erhält 28,5 g (96 % der Theorie) 5-Amino-1-(2,6-dichlor-4-trifluormethylphenyl)-pyrazol vom Schmelzpunkt 103° C-105° C.

Beispiel 2:

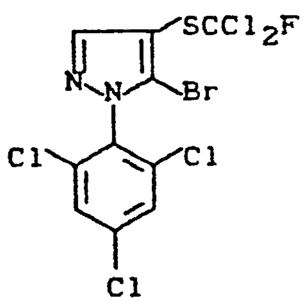

18,0 g (0,046 Mol) 5-Amino-4-dichlorfluormethylmercapto-1-(2,4,6-trichlorphenyl)-pyrazol werden in 60 ml Tribrommethan gelöst und bei Raumtemperatur mit 13,9 g (0,135 Mol) tert.-Butylnitrit versetzt. Die Temperatur steigt dabei auf 40-50° C an. Die Reaktionsmischung wird

vier Stunden nachgerührt, mit 200 ml Dichlormethan verdünnt und nacheinander mit Natriumhydrogencarbonat-, Natriumthiosulfat- und Kochsalzlösung gewaschen. Die organische Phase wird über Magnesiumsulfat getrocknet und im Vakuum vom Lösungsmittel befreit.

Man erhält 19,0 g (91 % der Theorie) an 5-Brom-4-dichlorfluormethansulfenyl-1-(2,4,6-trichlorphenyl)-pyrazol vom Schmelzpunkt 118° C-122° C.

Herstellung der Ausgangsverbindung

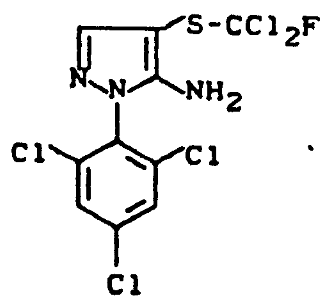

30 g (0,114 Mol) 5-Amino-1-(2,4-6-trichlorphenyl)-pyrazol und 10 ml (0,125 Mol) wasserfreies Pyridin in 150 ml Methylenchlorid werden bei 0° C bis 5° C mit 12,6 ml (0,12 Mol) Dichlorfluormethansulfenylchlorid versetzt und 30 Minuten gerührt. Zur Aufarbeitung gibt man 100 ml Methylenchlorid zu und wäscht nacheinander mit verdünnter Salzsäure, Wasser, Natriumcarbonatlösung und Kochsalzlösung, trocknet über Magnesiumsulfat und entfernt das Lösungsmittel im Vakuum.

Man erhält 44,5 g (98,7 % der Theorie) an 5-Amino-4-dichlorfluormethansulfenyl-1-(2,4,6-trichlorphenyl)-pyrazol vom Schmelzpunkt 101° C bis 106° C.

Beispiel 3:

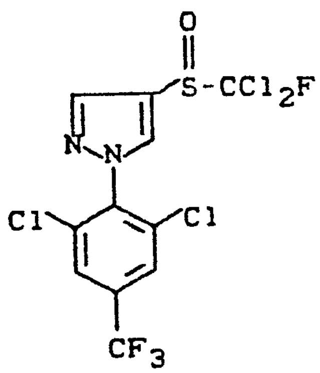

(I)

2,1 g (0,005 Mol) 4-Dichlorfluormethansulfenyl-1-(2,6-dichlor-4-trifluormethyl-phenyl)-pyrazol werden in 25 ml Dichlormethan gelöst und bei ca 10° C mit 1,2 g (0,007 Mol) 90 %iger m-Chlorperbenzoesäure versetzt. Man läßt die Temperatur innerhalb von 2 Stunden auf 20° C ansteigen und arbeitet danach folgendermaßen auf: Die ausgefallene m-Chlorbenzoesäure wird abfiltriert und mit wenig Dichlormethan gewaschen. Die vereinigten organischen Phasen werden mit Natriumhydrogencarbonat-, Natriumthiosulfat- und abermals Natriumhydrogencarbonatlösung gewaschen, über Magnesiumsulfat getrocknet und im Vakuum eingeengt.

Man erhält 1,9 g (87 % der Theorie) an 4-Dichlorfluormethansulfinyl-1-(2,6-dichlor-4-trifluormethyl-phenyl)-pyrazol vom Schmelzpunkt 144° C.

In entsprechender Weise und gemäß den allgemeinen Angaben zur Herstellung erhält man die folgenden 1-Aryl-pyrazole der allgemeinen Formel (I):

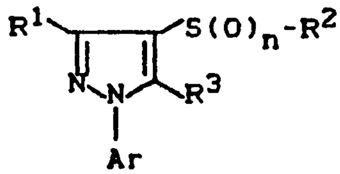

(I)

| Bsp. Nr. | $R^1$ | $R^2$ | $R^3$ | n | Ar | physikalische Daten |
|---|---|---|---|---|---|---|
| 4 | H | $CF_3$ | H | 0 | 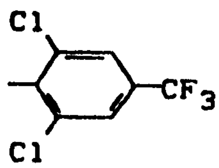 | $n_D^{20}$ = 1,4972 |
| 5 | H | $CCl_2F$ | Br | 0 | 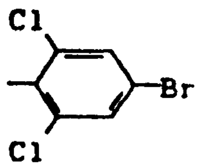 | Fp 118° C |
| 6 | H | $CCl_2F$ | Cl | 0 | 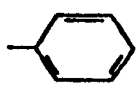 | Kp 150° C/ 0,13mbar |
| 7 | H | $CCl_2F$ | Cl | 0 | 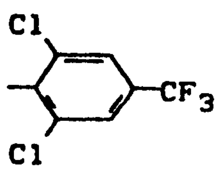 | Fp 172-174° C |
| 8 | H | $CCl_2F$ | Br | 0 | 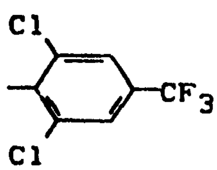 | Kp 145° C/ 0,013mbar |

| Bsp. Nr. | $R^1$ | $R^2$ | $R^3$ | n | Ar | physikalische Daten |
|---|---|---|---|---|---|---|
| 9 | H | $-CCl_2F$ | Br | 2 | 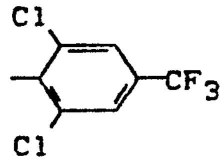 | Fp 104-108° C |
| 10 | H | $-CCl_2F$ | Br | 2 | 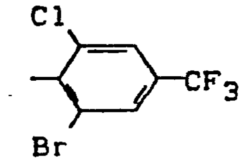 | Fp 78-82° C |
| 11 | H | $-CCl_2F$ | H | 0 | 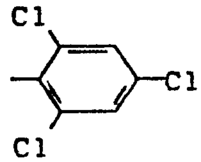 | $n_D^{20}$ = 1,6188 |
| 12 | H | $-CF_3$ | Br | 0 | 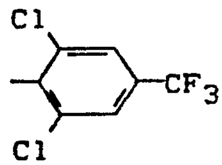 | $n_D^{20}$ = 1,5200 |
| 13 | H | $-CF_3$ | Br | 2 | 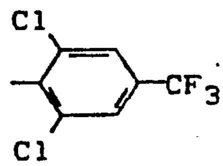 | Fp 90-91° C |
| 14 | $CH_3$ | $-CCl_2F$ | H | 0 | 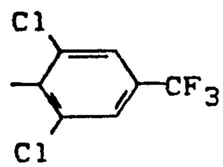 | 1H-NMR*) 7,81 ppm |
| 15 | $CH_3$ | $-CCl_2F$ | H | 0 | 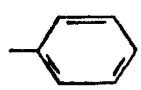 | $^1$H-NMR*) 8,1 ppm |

| Bsp. Nr. | $R^1$ | $R^2$ | $R^3$ | n | Ar | physikalische Daten |
|---|---|---|---|---|---|---|
| 16 | $CH_3$ | $-CCl_2F$ | Br | 0 | 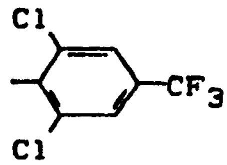 | Fp 60-62° C |
| 17 | $CH_3$ | $-CF_3$ | Br | 0 | 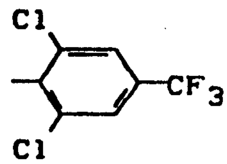 | Fp 68-70° C |
| 18 | $CH_3$ | $-CF_3$ | H | 0 | 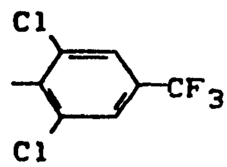 | $^1$H-NMR*) 7,76 ppm |
| 19 | H | $-CF_2Cl$ | Br | 0 | 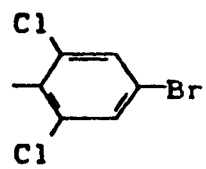 | Fp 99-102° C |
| 20 | H | $-CCl_2F$ | Br | 0 | 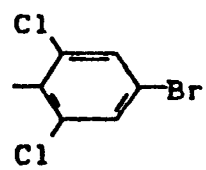 | Fp 115-118° C |

---

*) Die $^1$H-NMR-Spektren wurden in $CDCl_3$ mit Tetramethylsilan als innerem Standard aufgenommen. Angegeben ist die chemische Verschiebung (als δ-Wert) des Wasserstoffatoms in 5-Stellung des Pyrazolringes

| Bsp. Nr. | $R^1$ | $R^2$ | $R^3$ | n | Ar | physikalische Daten |
|---|---|---|---|---|---|---|
| 21 | $CH_3$ | $-CCl_2F$ | Cl | 0 | 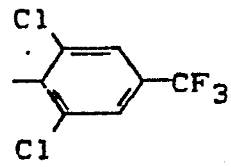 | Fp $63^{\circ}$ C |
| 22 | $CH_3$ | $-CF_3$ | Cl | 0 | 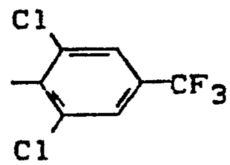 | Fp $59^{\circ}$ C |
| 23 | $CH_3$ | $-CF_3$ | H | 1 | 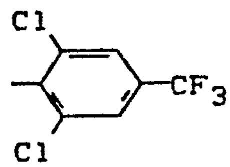 | Fp $81^{\circ}$ C |
| 24 | $CH_3$ | $-CCl_2F$ | H | 1 | 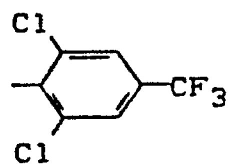 | Fp $100^{\circ}$ C |
| 25 | $CH_3$ | $-CF_3$ | Br | 2 | 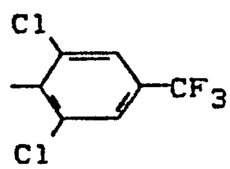 | Fp 84-$86^{\circ}$ C |
| 26 | $CH_3$ | $-CCl_2F$ | H | 2 | 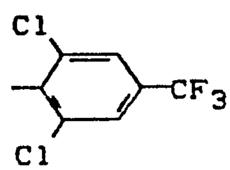 | Fp 88-$91^{\circ}$ C |
| 27 | H | $-CF_3$ | Br | 1 | 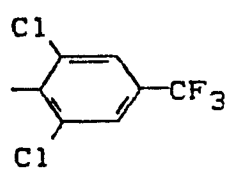 | Fp 94 $102^{\circ}$ C |

| Bsp. Nr. | $R^1$ | $R^2$ | $R^3$ | n | Ar | physikalische Daten |
|---|---|---|---|---|---|---|
| 28 | H | $-CF_3$ | Br | 0 | 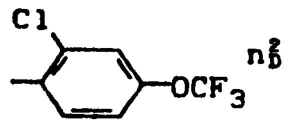 | $n_D^2=1,5010$ |
| 29 | H | $-CCl_2F$ | Br | 2 | 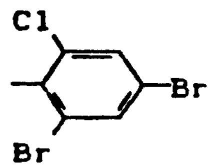 | Fp 125-130° C |
| 30 | H | $-CCl_2F$ | H | 0 | 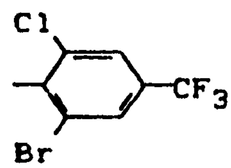 | zähes Öl |
| 31 | H | $-CCl_2F$ | Br | 1 | 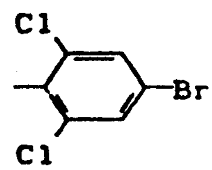 | Fp 113 122° C |
| 32 | H | $-CCl_2F$ | Br | 1 | 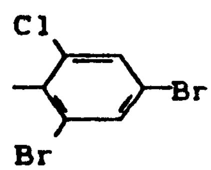 | Fp 148 153° C |
| 33 | H | $-CCl_2F$ | Br | 2 | 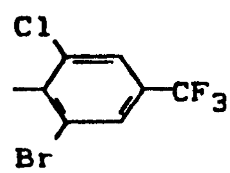 | Fp 97 110° C |
| 34 | H | $-CCl_2F$ | Br | 0 | 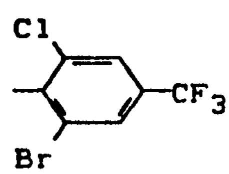 | Kp 180° C/ 0,05 mbar |

| Bsp. Nr. | $R^1$ | $R^2$ | $R^3$ | n | Ar | physikalische Daten |
|---|---|---|---|---|---|---|
| 35 | H | $-CCl_2F$ | Cl | 0 | 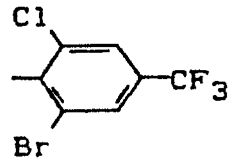 | Fp 183 185° C |
| 36 | H | $-CCl_2F$ | Br | 2 | 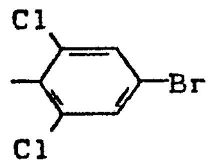 | Fp 114 122° C |
| 37 | H | $-CCl_2F$ | Br | 1 | 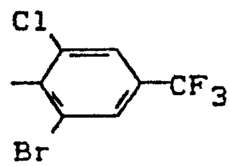 | Fp 122 134° C |
| 38 | H | $-CCl_2F$ | Br | 0 | 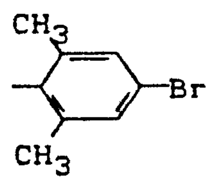 | Fp 87-92° C |
| 39 | H | $-CH_3$ | Br | 2 | 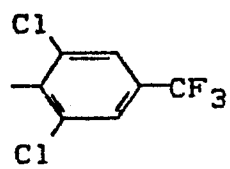 | Fp 130-132° C |
| 40 | $CH_3$ | $-CF_3$ | Br | 0 | 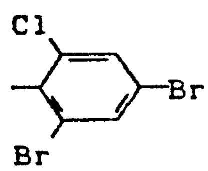 | Fp 101-102° C |

| Bsp. Nr. | $R^1$ | $R^2$ | $R^3$ | n | Ar | physikalische Daten |
|---|---|---|---|---|---|---|
| 41 | $C_2H_5$ | $-CCl_2F$ | Br | 0 | 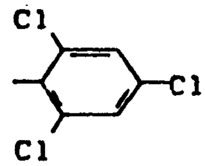 | $^1$H-NMR: δ=7,49ppm |
| 42 | $C_2H_5$ | $-CF_3$ | Br | 0 | 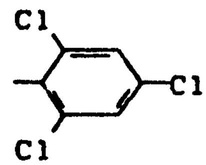 | $^1$H-NMR: δ-7,48ppm |
| 43 | H | $-CCl_2F$ | Br | 0 | 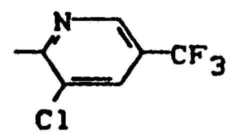 | Fp: 44-46° C |
| 44 | $CH_3$ | $-CCl_2F$ | Br | 1 | 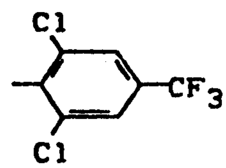 | Fp: 104-7° C |
| 45 | $CH_3$ | $-CF_3$ | Br | 1 | 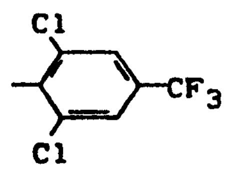 | Fp: 104° C |
| 46 | $CH_3$ | $-CF_3$ | H | 2 | 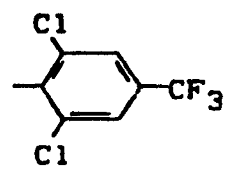 | Fp: 68-72° C |
| 47 | $CH_3$ | $-CCl_2F$ | Br | 0 | 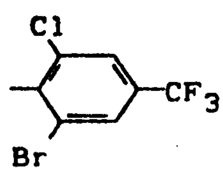 | Fp: 35-40° C |
| 48 | $CH_3$ | $-CF_3$ | Br | 0 | 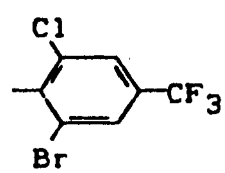 | Fp: 66-67° C |

| Bsp. Nr. | $R^1$ | $R^2$ | $R^3$ | n | Ar | physikalische Daten |
|---|---|---|---|---|---|---|
| 49 | $CH_3$ | $-CF_3$ | Br | 0 | 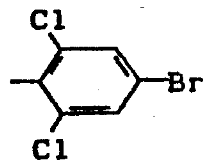 | Fp: 96-97° C |
| 50 | $CH_3$ | $-CClF_2$ | Br | 0 | 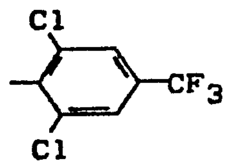 | Fp: 57-9° C |
| 51 | $CH_3$ | $-CCl_2F$ | Br | 0 | 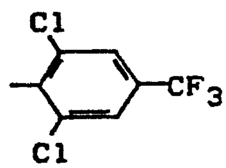 | Fp: 77-83° C |
| 52 | $CH_3$ | $-CF_3$ | Br | 0 | 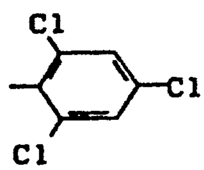 | Fp: 83-85° C |
| 53 | $CH_3$ | $-CCl_2F$ | Br | 0 | 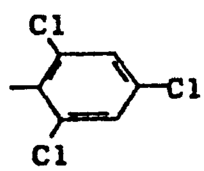 | Fp: 83-85° C |
| 54 | $CH_3$ | $-CClF_2$ | Br | 0 | 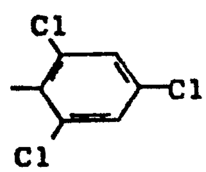 | Fp: 57-9° C |
| 55 | $CH_3$ | $-CF_3$ | Br | 0 | 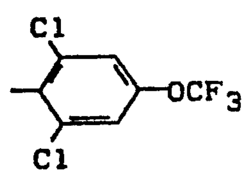 | Fp: 48-50° C |

0216102

| Bsp. Nr. | $R^1$ | $R^2$ | $R^3$ | n | Ar | physikalische Daten |
|---|---|---|---|---|---|---|
| 56 | $CH_3$ | $-CCl_2F$ | Br | 0 | 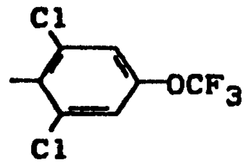 | Fp: 85-7° C |
| 57 | $CH_3$ | $-CClF_2$ | Br | 0 | 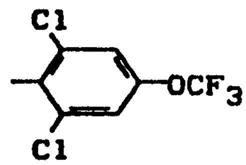 | Fp: 63-4° C |
| 58 | $CH_3$ | $-CF_3$ | Br | 2 | 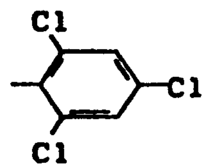 | Fp: 104-06° C |
| 59 | $CH_3$ | $-CCl_2F$ | Br | 0 | 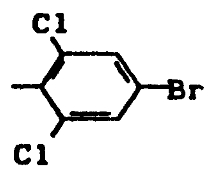 | Fp: 85-7° C |
| 60 | $CH_3$ | $-CCl_2F$ | Br | 2 | 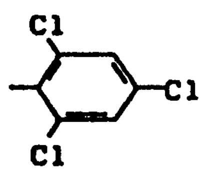 | $^1$H-NMR ($CDCl_3$): δ 2.56 (s, 3H), δ 7.50 (m, 2H) |
| 61 | $CH_3$ | $-CClF_2$ | Br | 2 | 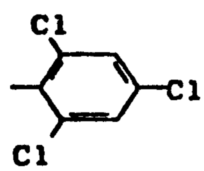 | Fp: 81-3° C |
| 62 | $CH_3$ | $-CF_3$ | Br | 2 | 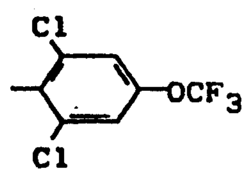 | Fp: 74-6° C |

| Bsp. Nr. | $R^1$ | $R^2$ | $R^3$ | n | Ar | physikalische Daten |
|---|---|---|---|---|---|---|
| 63 | $CH_3$ | $-CCl_2F$ | Br | 2 | 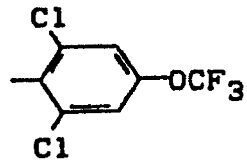 | $^1$H-NMR ($CDCl_3$): δ 2,60 (s, 3H) δ 7,37 (m, 2H) |
| 64 | $CH_3$ | $-CClF_2$ | Br | 2 | 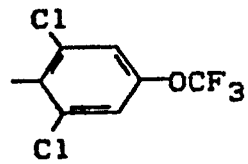 | Fp: 88-90° C |
| 65 | $CH_3$ | $-CClF_2$ | Br | 2 | 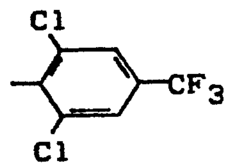 | Fp: 77-9° C |
| 66 | $CH_3$ | $-CClF_2$ | Br | 0 | 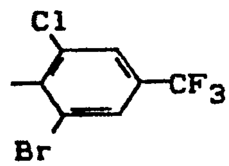 | Fp: 74-75° C |
| 67 | $CH_3$ | $-CCl_2F$ | Br | 0 | 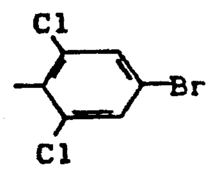 | Fp: 70-1° C |
| 68 | $CH_3$ | $-CClF_2$ | Br | 0 | 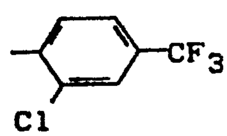 | Fp: 61-2° C |
| 69 | $CH_3$ | $-CClF_2$ | Br | 0 | 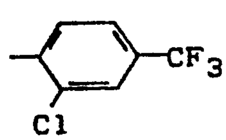 | Fp: 40-50° C $^1$H-NMR ($CDCl_3$) δ 2.47 (s,3H) |

| Bsp. Nr | $R^1$ | $R^2$ | $R^3$ | n | Ar | physikalische Daten |
|---|---|---|---|---|---|---|
| 70 | $CH_3$ | $-CF_3$ | Br | 0 | 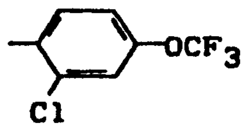 | Fp: 40-45° C<br>$^1$H-NMR ($CDCl_3$)<br>δ 2.45 (3H,s) |
| 71 | $CH_3$ | $-CCl_2F$ | H | 0 | 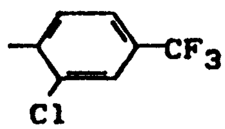 | $^1$H-NMR ($CDCl_3$)<br>δ 2.48 (s, 3H)<br>δ 7.68 (d.v.d, 1H)<br>δ 8.85 (d.v.d, 2H)<br>δ 8.29 (s, 1H) |
| 72 | $CH_3$ | $-CClF_2$ | H | 0 | 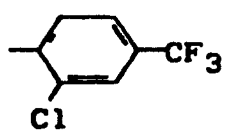 | $^1$H-NMR ($CDCl_3$)<br>δ 2.42 (s, 3H)<br>δ 7.69 (d.v.d, 1H)<br>δ 7.84 (2H)<br>δ 7,25 (s, 1H) |
| 73 | $CH_3$ | $-CF_3$ | H | 0 | 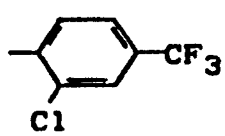 | $^1$H-NMR ($CDCl_3$)<br>δ 2.43 (s, 3H)<br>δ 7.67 (d.v.d, 1H)<br>δ 7.82 (m, 2H)<br>δ 8,24 (s, 1H) |
| 74 | H | $-CCl_2F$ | Br | 0 | 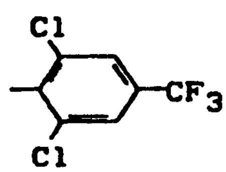 | $^1$H-NMR ($CDCl_3$)<br>δ 8.04 (s, 1H) |
| 75 | H | $-CCl_2F$ | H | 2 | 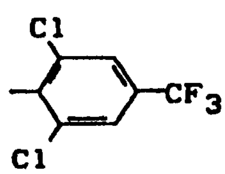 | Fp: 145-155° C |

| Bsp. Nr | $R^1$ | $R^2$ | $R^3$ | n | Ar | physikalische Daten |
|---|---|---|---|---|---|---|
| 76 | H | $-CF_3$ | Br | 0 | 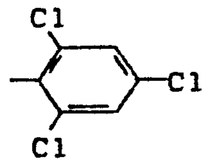 | Fp: 75-78° C |
| 77 | H | $-CF_3$ | Br | 0 | 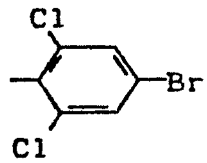 | Fp: 62-66° C |
| 78 | H | $-CCl_2F$ | Br | 1 | 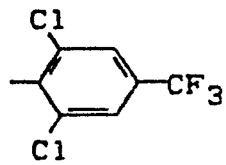 | Fp: 125-28° C |
| 79 | H | $-CClF_2$ | H | 0 | 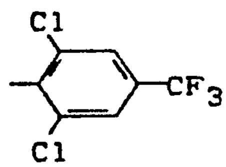 | $^1$H-NMR ($CDCl_3$)<br>δ 7.87 (s, 1H)<br>δ 7.98 (s, 1H) |
| 80 | H | $-CF_3$ | H | 1 | 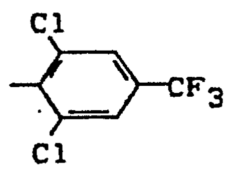 | Fp: 102-03° C |
| 81 | H | $-CClF_2$ | H | 1 | 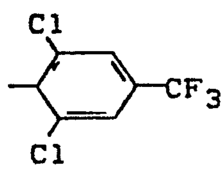 | Fp: 126-27° C |
| 82 | H | $-CF_3$ | Br | 0 | 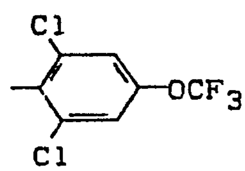 | $n_D^{20}$ : 1,5090 |

| Bsp. Nr | $R^1$ | $R^2$ | $R^3$ | n | Ar | physikalische Daten |
|---|---|---|---|---|---|---|
| 83 | H | $-CF_3$ | H | 2 | 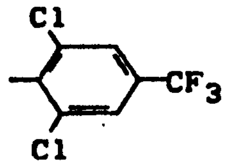 | Fp: 108-09° C |
| 84 | H | $-CCl_2F$ | Br | 0 | 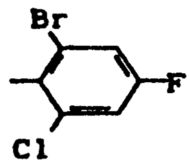 | ¹H-MNR ($CDCl_3$) δ 7.98 (s,1H) |
| 85 | H | $-CF_2Cl$ | Br | 0 | 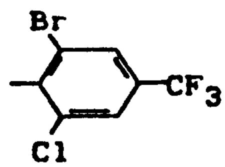 | ¹H-NMR ($CDCl_3$) δ 8.03 (s, 1H) |
| 86 | H | $-CClF_2$ | Br | 0 | 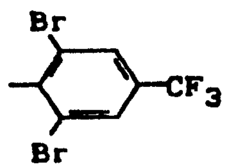 | Kp: 175° C/0,05 mbar |
| 87 | H | $-CClF_2$ | Br | 2 | 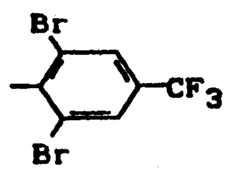 | Fp: 90-100° C |
| 88 | H | $-CClF_2$ | Br | 0 | 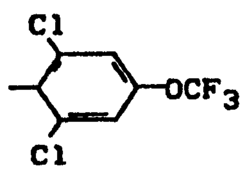 | Kp: 160° C/0,01 mbar |
| 89 | H | $-CCl_2F$ | Br | 0 | 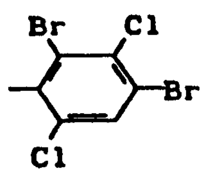 | Fp: 124-27° C |

| Bsp. Nr | $R^1$ | $R^2$ | $R^3$ | n | Ar | physikalische Daten |
|---|---|---|---|---|---|---|
| 90 | H | $-CCl_2F$ | Br | 0 | 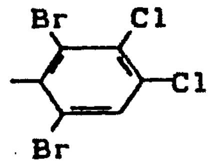 | Fp: 116-20° C |
| 91 | H | $-CCl_2F$ | Br | 2 | 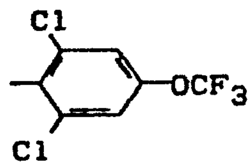 | Fp: 57-61° C |
| 92 | H | $-CCl_2F$ | Br | 1 | 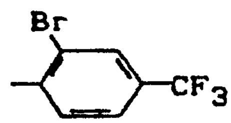 | Fp: 110-120° C |
| 93 | H | $-CClF_2$ | Br | 1 | 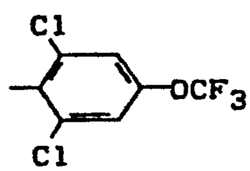 | Fp: 92-95° C |
| 94 | H | $-CCl_2F$ | Br | 2 | 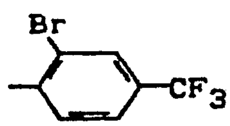 | $^1$H-NMR ($CDCl_2$) δ 8.25 (s, 1H) |
| 95 | H | $-CCl_2F$ | Br | 0 | 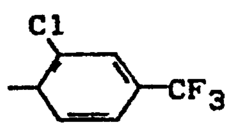 | Kp: 150° C/0,02 mbar |
| 96 | H | $-CF_3$ | H | 0 | 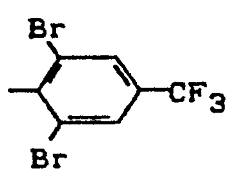 | $^1$H-NMR ($CDCl_3$) δ 7.76 (d, 1H) δ 7.88 (d, 3H) |

| Bsp. Nr | $R^1$ | $R^2$ | $R^3$ | n | Ar | physikalische Daten |
|---|---|---|---|---|---|---|
| 97 | H | $-CCl_2F$ | H | 0 | 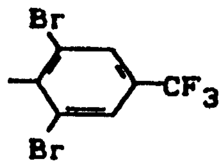 | Fp: 69-71° C |
| 98 | H | $-CF_2Cl$ | Br | 2 | 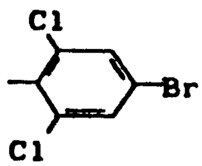 | Fp: 90-39° C |
| 99 | H | $-CF_2Cl$ | H | 0 | 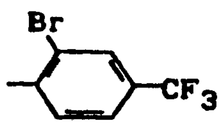 | Kp: 140° C/0,01 mbar |
| 100 | H | 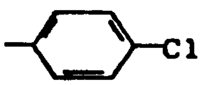 | Br | 2 | 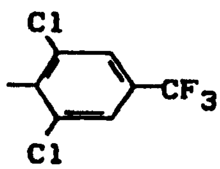 | Fp: 164-67° C |
| 101 | H | $-CH_3$ | H | 0 | 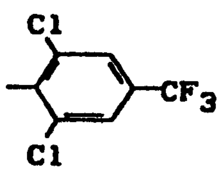 | $n_D^{20}$ : 1,5525 |
| 102 | $CH_3$ | $-CH_3$ | H | 0 | 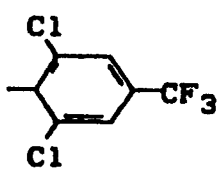 | $n_D^{20}$ : 1,5519 |

0216102

Anwendungsbeispiele:

In den folgenden Anwendungsbeispielen wurden die nachstehend aufgeführten Verbindungen als Vergleichsubstanzen eingesetzt:

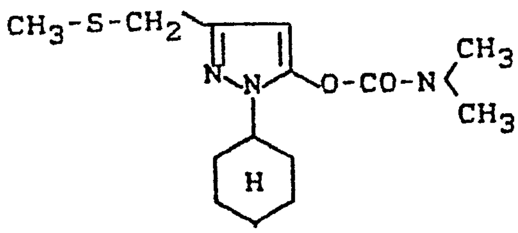

(A)

1-Cyclohexyl-5-[N,N-(dimethyl)-carbamoyloxy]-3-methylthiomethyl-pyrazol
(bekannt aus DE-OS 2 839 270)

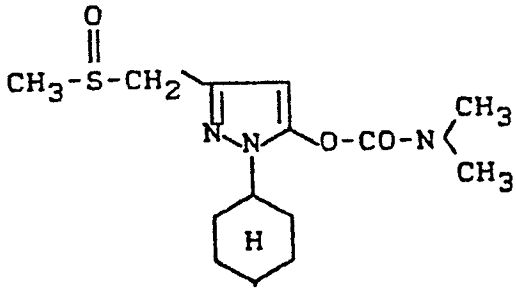

(B)

1-Cyclohexyl-5-[N,N-(dimethyl)-carbamoyloxy]-3-methylsulfinylmethyl-pyrazol
(bekannt aus DE-OS 2 839 270)

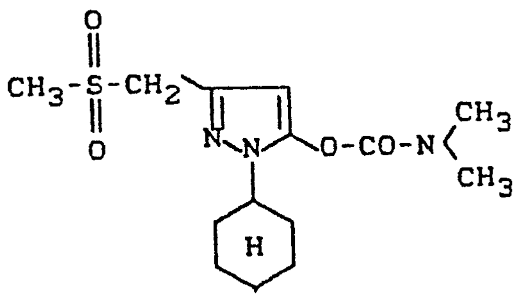

(C)

1-Cyclohexyl-5-[N,N-(dimethyl)-carbamoyloxy]-3-methylsulfonylmethyl-pyrazol (bekannt aus DE-OS 2 839 270)

Beispiel A

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 5, 7, 11, 14, 16, 4, 28, 12, 18, 17, 40, 27, 13, 39, 37, 10, 36, 29, 2, 19, 34, 20, 35, 46.

Beispiel B

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 5, 14, 38, 2, 34, 10, 20, 36.

0216102

Beispiel C

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 14, 16.

Beispiel D

$LT_{100}$-Test für Dipteren

Testtiere: Musca domestica
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^3$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1, 8, 14, 16, 46, 79, 83, 102, 17, 18, 22, 21, 24, 45, 25, 48, 50, 55, 56, 65, 66, 70, 71, 73, 12, 13, 4, 74, 27, 81, 82, 34, 30, 85, 88, 95, 97, 99, 101, 80, 96, 23.

Beispiel E

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^3$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1, 7, 8, 11, 14, 16, 49, 79, 83, 102, 17, 18, 22, 21, 23, 24, 45, 25, 46, 48, 50, 55, 56, 59, 62, 65, 66, 70, 71, 73, 12, 13, 4, 74, 27, 81, 82, 9, 10, 33, 34, 37, 30, 85, 88, 95, 97, 99, 101, 80, 96.

Beispiel F

$LD_{100}$-Test

Testtiere: Leucophaea maderae
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1, 8, 14, 46, 79, 83, 102.

Beispiel G

Test mit Lucilia cuprina res.-Larven
(OP-res. Goondiwindi-Stamm)

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 8, 14.

Beispiel H

Test mit parasitierenden, adulten Stechfliegen (Stomoxys calcitrans)

Lösungsmittel: Cremophor

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Stechfliegen (Stomoxys calcitrans) werden in Petrischalen, die Sandwiches enthalten, die einen Tag vor Versuchsbeginn mit 1 ml der zu testenden Wirkstoffzubereitung durchtränkt wurden, gebracht. Nach 3 Stunden wird der Abtötungsgrad in Prozent bestimmt, wobei 100 % bedeuten, daß alle und 0 %, daß keine Fliege abgetötet worden sind .

Bei diesem Test zeigen z.B. die folgenden Verbindungen der Herstellungsbeipiele überlegene Wirkung gegenüber dem Stand der Technik: 1 und 7.

Beispiel I

$LD_{100}$-Test

Testtiere: Blatella germanica
Lösungsmittel: Aceton

2 Gewichtsteile Wirkstoff werden in 1000 Volumenteilen Lösungsmittel aufgenommen. Die so erhaltene Lösung wird mit weiteren Lösungsmittel auf die gewünschten Konzentrationen verdünnt.

2,5 ml Wirkstofflösung werden in eine Petrischale pipettiert. Auf dem Boden der Petrischale befinden sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 10 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Hierbei zeigen beispielsweise die Wirkstoffe aus folgenden Beispielen bei einer Wirkstoffkonzentration von höchstens 0,2 % in der Testlösung eine 100 %ige Abtötung:
18, 22, 21, 23, 45, 25, 46, 48, 50, 55, 62, 65, 66, 71, 73, 12, 4, 74, 27, 79, 82, 83, 34, 30, 85, 88, 95, 97, 99, 101, 102, 80, 96.

0216102

Patentansprüche

1. 1-Aryl-pyrazole der allgemeinen Formel

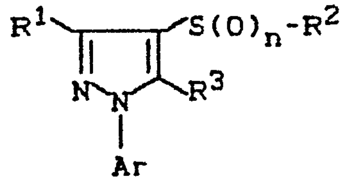

(I)

in welcher

$R^1$ für Wasserstoff, Alkyl oder Halogenalkyl steht,

$R^2$ für Alkyl, Halogenalkyl oder für jeweils gegebenenfalls substituiertes Aralkyl oder Aryl steht,

$R^3$ für Wasserstoff oder Halogen steht,

Ar für gegebenenfalls substituiertes Phenyl oder Pyridyl steht,

und

n für eine Zahl 0,1 oder 2 steht.

2. 1-Aryl-pyrazole der Formel (I) gemäß Anspruch 1, dadurch gekennzeichnet, daß

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen steht oder für jeweils gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Phenylsubstituenten jeweils in Frage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für Wasserstoff, Fluor, Chlor, Brom oder Iod steht,

Ar für gegebenenfalls einfach oder mehrfach, gleich oder verschieden substituiertes Phenyl, 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$,

wobei

$R^4$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

m für eine Zahl 0, 1 oder 2 steht und

n für eine Zahl 0, 1 oder 2 steht.

3. 1-Aryl-pyrazole der Formel (I) gemäß Anspruch 1, dadurch gekennzeichnet, daß

$R^1$ für Wasserstoff, Methyl, Ethyl, n- oder i-Propyl und Trifluormethyl steht,

$R^2$ für Methyl, Ethyl, n- oder i-Propyl, n-, i-, s- oder t-Butyl, n- oder i-Pentyl, n- oder i-Hexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl oder für jeweils gegebenenfalls einfach bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Wasserstoff, Fluor, Chlor oder Brom steht,

Ar für gegebenenfalls ein- bis fünffach, gleich oder verschieden substituiertes Phenyl oder für jeweils gegebenenfalls ein- bis vierfach gleich oder verschieden substituiertes 2-Pyridyl oder 4-Pyridyl steht, wobei als Phenyl- bzw. Pyridylsubstituenten jeweils in Frage kommen: Cyano, Nitro, Fluor, Chlor, Brom, Iod, Methyl, Ethyl, n- und i-Propyl, n-, i-, s- und t-Butyl,

Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$,

wobei

$R^4$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht,

m für eine Zahl 0, 1 oder 2 steht und

n für eine Zahl 0, 1 oder 2 steht.

4. Verfahren zur Herstellung von 1-Aryl-pyrazolen der allgemeinen Formel

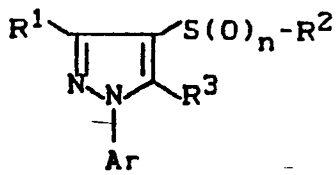

(I)

in welcher

$R^1$ für Wasserstoff, Alkyl oder Halogenalkyl steht,

$R^2$ für Alkyl, Halogenalkyl oder für jeweils gegebenenfalls substituiertes Aralkyl oder Aryl steht,

$R^3$ für Wasserstoff oder Halogen steht,

Ar für gegebenenfalls substituiertes Phenyl oder Pyridyl steht,

und

n für eine Zahl 0, 1 oder 2 steht,

dadurch gekennzeichnet, daß man 5-Amino-1-aryl-pyrazole der Formel (II),

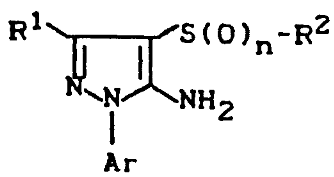

(II)

in welcher

$R^1$, $R^2$, Ar und n die oben angegebene Bedeutung haben,

mit einem anorganischen oder organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

5. Schädlingsbekämpfungsmittel, gekennzeichnet durch einen Gehalt an mindestens einem 1-Aryl-pyrazol der Formel (I).

6. Insektizide und akarizide Mittel, gekennzeichnet durch einen Gehalt an mindestens einem 1-Aryl-pyrazol der Formel (I).

7. Verfahren zur Bekämpfung von Insekten und/oder Spinnentieren und/oder Nematoden,

dadurch gekennzeichnet, daß man 1-Aryl-pyrazole der Formel (I) auf Insekten und/oder Spinnentiere und/oder Nematoden und/oder deren Lebenraum einwirken läßt.

8. Verwendung von 1-Aryl-pyrazolen der Formel (I) zur Bekämpfung von Insekten und/oder Spinnentieren und/oder Nematoden.

9. Verfahren zur Herstellung von Schädlingsbekämpfungsmitteln, dadurch gekennzeichnet, daß man 1-Aryl-pyrazole der Formel (I) mit Streckmitteln und/oder oberflächenaktiven Mitteln vermischt.

0216102

Europäisches Patentamt

# EUROPÄISCHER RECHERCHENBERICHT

Nummer der Anmeldung

EP 86 11 1075

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | EP-A-0 138 527 (NIPPON KAYAKU K.K.)<br>* Tabelle II, Verbindungen Nr. 144-146 *<br>--- | 1-3 | C 07 D 401/04<br>C 07 D 231/18<br>A 01 N 43/56 //<br>C 07 D 231/44<br>C 07 D 231/38 |
| X | EP-A-0 025 859 (BASF AG)<br>* Beispiele *<br>--- | 4 | |
| X | EP-A-0 151 866 (ELI LILLY AND CO.)<br>* Seite 9, Zeile 1 - Seite 10, Zeile 26, Beispiele 1B, 2B, 3B *<br>--- | 4 | |
| A | GB-A-2 073 172 (SHELL INTERNATIONALE RESEARCH MAATSCHAPPIJ)<br>* Ansprüche 1, 9, 10 *<br>--- | 1,5-9 | RECHERCHIERTE SACHGEBIETE (Int Cl 4) |
| A | DE-A-2 409 753 (BASF AG)<br>* Anspruch 1 *<br>--- | 1 | C 07 D 401/00<br>C 07 D 231/00<br>A 01 N 43/00 |
| A,D | DE-A-2 839 270 (BAYER AG)<br>----- | | |

Der vorliegende Recherchenbericht wurde für alle Patentansprüche erstellt.

| Recherchenort | Abschlußdatum der Recherche | Prüfer |
|---|---|---|
| BERLIN | 28-11-1986 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82